# EUROPEAN PATENT APPLICATION

(11) **EP 2 384 714 A1**
(43) Date of publication of application: **09.11.2011**
(21) Application number: 10382105.4
(22) Date of filing: 03.05.2010
(51) Int. Cl.: A61B 19/00

(54) **A method for defining working space limits in robotic surgery**

(71) Applicant: Universitat Politècnica de Catalunya, 08034 Barcelona (ES)
(72) Inventor: Amat Girbau, Josep, 08023, Barcelona (ES); Casals Gelpi, Alicia, 08960, Sant Just Desvern (ES); Frigola Bourlon, Manel, 17300, Blanes (ES)
(74) Representative: ZBM Patents

(57) **Abstract**

A method for defining working space limits in robotic surgery, wherein a user manages a surgical robot acting on at least one anatomical element, the movements of the surgical robot being to be limited within said working space limits, the method comprising: representing a three dimensional image of the anatomical element in a virtual three dimensional space; representing at least one plane intersecting the three dimensional image of the anatomical element in the virtual three dimensional space; and representing the working space limits according to the space being limited by said at least one plane intersecting the three dimensional image of the anatomical element in the virtual three dimensional space.

## Description

The present invention relates to a method for defining working space limits in robotic surgery, wherein a user manages a surgical robot acting on at least one anatomical element, the movements of the surgical robot being to be limited within said working space limits.

The invention also relates to a system, a Graphical User Interface (GUI) and a computer program for defining working space limits in robotic surgery suitable for carrying out such a method.

### BACKGROUND ART

For some surgical procedures, robotic aids may represent the difference between success and failure, reducing the surgeon stress produced by critical operations and improving general performance. For example, some methodologies are mainly focused on procedures involving bone milling. Such procedures are very frequent in many surgical specialties such as orthopedics, neurosurgery, otolaryngology and cranio-maxillofacial surgery. The prevalence of such surgery motivates research in this area. For example in a cochlear implant a space must be milled in the skull in order to fit the electronics. This space must be minimized and must match with the shape of the hardware as much as possible.

Another kind of surgery where robotic assistance could help is bone reconstruction, that is a piece of patient's bone must be harvested from a bone and then used as a "growth seed" in another part of his/her skeleton. In this case, the size and the shape of the extracted piece could be optimized by computational methods. In the same way, robotics can help in all surgeries that involve milling the space where a prosthesis must fit, for instance in knee replacement surgeries. A very illustrative example is osteoarthritis in the inner part of the cervical vertebras. People suffering this disease have an overgrowth of the internal part of the vertebra and, consequently, they may loss mobility below this vertebra.

Milling of the bone excess and release of the spinal cord compression is necessary in these cases. Corpectomy is the name of this procedure and consists in milling this excess of bone going through the vertebral body. In order to reach this area the surgeon must open his own path through the mouth of the patient (in fact above the mouth, between the mouth and the nose in order to not harm the inner part of the mouth). When the path is opened the surgeon must mill all the "sane" part of the vertebra in order to remove de overgrowth volume. Once this bone is milled the pressure over the spinal cord will decrease and the nerves will be able to work as in a sane patient.

The risk in this intervention is extremely high because any error must become an injury of the spinal cord and, consequently, the patient would become an incurable paraplegic. The sacrifice of part of the vertebra is necessary to reach the area of interest. In this kind of procedures putting a robot between surgeon and patient must give some extra intra-surgery information, provide precise presurgical planning and execution and minimize the amount of sacrificed bone to generate an access. Using a comanipulation environment as a navigator, it is possible to see where the milling end effector is in reference to the body corresponding CT (Computerized Tomography) image. Then the surgeon should be able to operate using a monitor as reference.

It is also possible to provide aids such as tremor reduction or scale magnifying/reduction if needed. Another performance that can be achieved easily by using a robot is the limitation of cutting speed. In some surgeries the velocity must be limited in order to not overheat the bone while cutting it. If this happens the cells on the bone dies and the bone is not able to recover itself. The most important contribution that a robot can provide to this surgery is, in fact, the addition of virtual fixtures to the environment.

The surface of the bone to be milled can be extracted from a medical image (it has an organic and complex 3D surface), we can add this surface as a virtual protection to our environment. In this way, the surgeon will be able to mill all along the workspace of the robot knowing that he will never touch the spinal cord that is in the other side of the virtual protection.

Graphical interfaces are very usual in different fields wherein human-computer interaction is fundamental, for example: robotic surgery. The main drawback of the known computer implemented interfaces and underlying methods is complexity for controlling the diverse parameters defining simple planes or three dimensional surfaces in the space, with the aim of configuring or adjusting the design of said three dimensional geometric structures according to the requirements of the user. Said geometric structures may be used, for example, for defining working space limits in robotic surgery.

The usual three dimensional graphic systems comprises user tools or functionalities for carrying out basic operations of translating, rotating, enlarging/reducing, etc. volumetric elements, normally by locating and dragging through the mouse an imaginary or indicated point of a cube or sphere surrounding said volumetric element.

For example, CAD (Computer-aided design) applications are an important industrial art extensively used in many applications, including automotive, shipbuilding, and aerospace industries, industrial, architectural design, etc. Due to CAD applications are general purpose applications, a lot of parameters are taken into account when representing geometric elements and very complex functionalities are available for defining said parameters. Thus, CAD applications do not have a good usability for defining working space limits in robotic surgery, since surgeons should deal with complex parameters and related processes, so that performance of the surgeon during interventions is deficient.

Specific graphical applications for defining working space limits in surgical robotics are not known in the state of the art. Nevertheless, some applications having some kind of relation with said objective are known. For instance, the US patent application US 2008/0005212 A1 discloses a system of curve generation that could help surgeons for defining working space limits. However, said system has the drawbacks of not comprising 3D spaces representation and being very complex because a lot of parameters and not ease-of-use functionalities for managing said parameters. More precisely, the system of curve generation takes a sequence of control points and constraint codes for each control point, and outputs a curve in which each of the constraints is satisfied. The set of constraints is chosen from the tangent angle, curvature, first derivative of curvature, and second derivative of curvature. The interactive curve design uses as its primitive, a curve whose curvature is a polynomial function of arclength (whose intrinsic equation is a polynomial). At each control point, a choice of G2 curvature continuity (tangent angle and curvature) or G4 curvature continuity (tangent angle and curvature plus first and second derivatives of curvature are continuous) is input. The desired curve is expressed as the solution to the chosen set of constraints.

The US patent application US 2007/0253617 A1 discloses a method and apparatus for reconstructing a surface shape of an object having contour lines that could be of application for defining working limits in robotic surgery. Said method and apparatus includes assigning points to each contour line. A first triangulation scheme is performed with respect to respective points on two adjacently positioned contour lines, to determine a first surface shape for a portion of the object corresponding to the two contour lines. The first surface shape is checked to determine if the first surface shape is in error. If the first surface shape is not in error, the first surface shape is outputted for the portion of the object as determined by the first triangulation scheme, as a reconstructed surface shape for that portion of the object. If the first surface shape is in error, a second triangulation scheme is performed with respect to the respective points on the two contour lines, to determine a second surface shape for that portion of the object, and the second surface shape is outputted for that portion of the object as determined by the second triangulation scheme, as a reconstructed surface shape. Therefore, in this case, the drawback of considering a lot of parameters and not ease-of-use functionalities for managing said parameters still remains.

The US patent US 6,639,592 B1 discloses a method of modeling complex surface models using a network of intersecting non-uniform rational B-spline curves. Topological information of the curve network and interpolating surfaces to the network of curves are automatically generated. Different levels of continuity between surface patches are enforced. Surface patches of three and four sides and positional, tangent or curvature continuity between the patches are provided. Using a constrained minimization process, arbitrary, non-uniform B-spline curves may be used to manipulate the shape of the surfaces interpolating the curve network without violating the continuity conditions enforced during the generation of the surface patches allowing for very complex three-dimensional shapes to be modeled using the method. In this other case, the drawback of considering a lot of parameters and not ease-of-use functionalities for managing said parameters is also present.

### SUMMARY OF THE INVENTION

There thus still exists a need for a new method (and related computer program, system and graphical user interface) for defining working space limits in robotic surgery, wherein a user manages a surgical robot acting on at least one anatomical element, the movements of the surgical robot being to be limited within said working space limits, said method (and related computer program, system and graphical user interface) improving usability and performance during surgical interventions in comparison with the known methods (and related computer programs, systems and graphical user interfaces) in the state of the art.

The object of the present invention is to fulfil such a need. Said object is achieved with a method for defining working space limits in robotic surgery according to claim 1, a system for defining working space limits in robotic surgery according to claim 13, a Graphical User Interface for defining working space limits in robotic surgery according to claim 14 and a computer program product comprising program instructions for causing a computer to perform said method according to claim 15.

In a first aspect, the present invention provides a method of the mentioned type comprising:
- representing a three dimensional image of the anatomical element in a virtual three dimensional space;
- representing at least one plane intersecting the three dimensional image of the anatomical element in the virtual three dimensional space;
- representing the working space limits according to the space being limited by said at least one plane intersecting the three dimensional image of the anatomical element in the virtual three dimensional space.

This method only comprises three simple steps for defining working space limits in robotic surgery, so very few intuitive user actions are necessary for accomplish said objective. Then, the method of the invention offers ease-of-use facilities that make easier for the surgeon defining working space limits in robotic surgery and, therefore, performance of robotic surgical operations is highly improved.

Preferably, representing at least one plane intersecting the three dimensional image of the anatomical element in the virtual three dimensional space comprises:
- if a user request for representing at least one curve segment in the virtual three dimensional space is detected:
   - representing said at least one curve segment in the virtual three dimensional space according to the user request for representing said at least one curve segment;
- if a user request for representing at least one of the planes intersecting the three dimensional image of the anatomical element from said at least one curve segment in the virtual three dimensional space is detected:
   - representing said one of the planes intersecting the three dimensional image of the anatomical element from said at least one curve segment in the virtual three dimensional space.

Representing a plane from just a curve segment also implies that very few intuitive user actions are necessary for that. Then, a further ease-of-use facility for the surgeon is offered and, therefore, performance of robotic surgical operations is highly improved.

In preferred embodiments of the invention, the user request for representing at least one curve segment in the virtual three dimensional space comprises:
- a user request for representing a plurality of control points in the virtual three dimensional space;
- a user request for representing said curve segment from the plurality of control points in the virtual three dimensional space;
and representing said at least one curve segment in the virtual three dimensional space according to the user request for representing said at least one curve segment comprises:
- representing the plurality of control points in the virtual three dimensional space according to the user request for representing the plurality of control points;
- representing said curve segment by polynomial interpolation of the plurality of control points in the virtual three dimensional space according to the user request for representing said curve segment from the plurality of control points.

Polynomial interpolation is not described in this patent application because it is a well known method in the field of graphics and details of said method are not an object of the present invention.

Representing a curve segment from only (at least) two control points also implies that very few intuitive user actions are necessary for that. Then, a further ease-of-use facility for the surgeon is offered and, therefore, performance of robotic surgical operations is highly improved.

In some embodiments of the invention, the user request for representing at least one curve segment in the virtual three dimensional space comprises:
- if the user request refers to rotation of said curve segment:
   - a user request for representing at least one of the control points as a rotation centre point in the virtual three dimensional space;
   - a user request for rotating the curve segment by moving one of the control points respect to the rotation centre point;
and representing said at least one curve segment in the virtual three dimensional space according to the user request for representing said at least one curve segment comprises:
- if the user request refers to rotation of said curve segment:
   - representing the rotation centre point in the virtual three dimensional space according to the user request for representing the control point as the rotation centre point;
   - representing the curve segment being rotated by moving one of the control points respect to the rotation centre point according to the user request for rotating the curve segment.

Rotating a curve segment by defining a rotation centre point and dragging one of the other control points respect to said rotation centre point also implies that very few intuitive user actions are necessary for that. Then, a further ease-of-use facility for the surgeon is offered and, therefore, performance of robotic surgical operations is highly improved. Moreover, said rotating functionality helps the user adjusting the configuration of curve segments in the virtual 3D space, so that representations of very high accuracy can be achieved.

In a preferred embodiment of the invention, the user request for representing at least one curve segment in the virtual three dimensional space comprises:
- if the user request refers to flexion of said curve segment:
   - a user request for representing at least one of the control points as a flexing centre point in the virtual three dimensional space;
   - a user request for flexing the curve segment by moving at least one of the control points respect to the flexing centre point;
and representing said at least one curve segment in the virtual three dimensional space according to the user request for representing said at least one curve segment comprises:
- if the user request refers to flexion of said curve segment:
   - representing the flexing centre point in the virtual three dimensional space according to the user request for representing the control point as the flexing centre point;
   - representing the curve segment being flexed by moving at least one of the control points respect to the flexing centre point according to the user request for flexing the curve segment.

Flexing a curve segment by defining a flexing centre point and dragging at least one of the other control points respect to said flexing centre point also implies that very few intuitive user actions are necessary for that. Then, a further ease-of-use facility for the surgeon is offered and, therefore, performance of robotic surgical operations is highly improved. Moreover, said flexing functionality helps the user adjusting the configuration of curve segments in the virtual 3D space, so that representations of very high accuracy can be achieved.

In some embodiments, the user request for representing at least one of the planes intersecting the three dimensional image of the anatomical element from said at least one curve segment in the virtual three dimensional space comprises:
- if the user request refers to representation of plane by translation of curve segment:
   - a user request for representing said one of the planes intersecting the three dimensional image of the anatomical element by translation in a predetermined direction of said curve segment in the virtual three dimensional space;
and wherein representing said one of the planes intersecting the three dimensional image of the anatomical element from said at least one curve segment in the virtual three dimensional space comprises:
- if the user request refers to representation of plane by translation of curve segment:
   - representing said one of the planes intersecting the three dimensional image of the anatomical element by translation in a predetermined direction of said curve segment in the virtual three dimensional space.

Representing a plane intersecting the three dimensional image of the anatomical element by translation in a predetermined direction of a curve segment also implies that very few intuitive user actions are necessary for that. Then, a further ease-of-use facility for the surgeon is offered and, therefore, performance of robotic surgical operations is highly improved.

In preferred embodiments, the user request for representing at least one of the planes intersecting the three dimensional image of the anatomical element from said at least one curve segment in the virtual three dimensional space comprises:
- if the user request refers to representation of plane from at least two curve segments:
   - a user request for representing said one of the planes intersecting the three dimensional image of the anatomical element from at least two curve segments of the at least one curve segment in the virtual three dimensional space;
and representing said one of the planes intersecting the three dimensional image of the anatomical element from said at least one curve segment in the virtual three dimensional space comprises:
- if the user request refers to representation of plane from at least two curve segments:
   - representing said one of the planes intersecting the three dimensional image of the anatomical element by parametric extension of said at least two curve segments of the at least one curve segment in the virtual three dimensional space.

Parametric extension of curve segments is not described in this patent application because it is a well known method in the field of graphics and details of said method are not an object of the present invention.

Representing a plane intersecting the three dimensional image of the anatomical element by parametric extension of said at least two curve segments also implies that very few intuitive user actions are necessary for that. Then, a further ease-of-use facility for the surgeon is offered and, therefore, performance of robotic surgical operations is highly improved. Moreover, said parametric extension based functionality helps the user adjusting the configuration of planes in the virtual 3D space, so that representations of very high accuracy can be achieved.

Preferably, representing at least one plane intersecting the three dimensional image of the anatomical element in the virtual three dimensional space comprises:
- if a user request for representing at least one of the planes intersecting the three dimensional image of the anatomical element from a previously represented plane in the virtual three dimensional space is detected:
   - representing said one of the planes intersecting the three dimensional image of the anatomical element by replication of said previously represented plane in the virtual three dimensional space.

Representing a plane intersecting the three dimensional image of the anatomical element by replication of a previously represented plane also implies that very few intuitive user actions are necessary for generating planes. Then, a further ease-of-use facility for the surgeon is offered and, therefore, performance of robotic surgical operations is highly improved.

In preferred embodiments of the invention, representing at least one plane intersecting the three dimensional image of the anatomical element in the virtual three dimensional space comprises:
- if a user request for representing a point comprised in a previously represented plane as a rotation centre point of the plane in the virtual three dimensional space is detected:
   - representing said point comprised in the previously represented plane as a rotation centre point of the plane in the virtual three dimensional space;
- if a user request for rotating the previously represented plane by moving at least one of the points comprised in the previously represented plane respect to the rotation centre point is detected:
   - representing said previously represented plane being rotated by moving at least one of the points comprised in said previously represented plane respect to the rotation centre point.

Rotating a plane by defining a rotation centre point and dragging at least one of the other control points respect to said rotating centre point also implies that very few intuitive user actions are necessary for that. Then, a further ease-of-use facility for the surgeon is offered and, therefore, performance of robotic surgical operations is highly improved. Moreover, said rotating functionality helps the user adjusting the configuration of planes in the virtual 3D space, so that representations of very high accuracy can be achieved.

In some embodiments of the invention, representing at least one plane intersecting the three dimensional image of the anatomical element in the virtual three dimensional space comprises:
- if a user request for representing a point comprised in a previously represented plane as a flexing centre point of the plane in the virtual three dimensional space is detected:
   - representing said point comprised in the previously represented plane as a flexing centre point of the plane in the virtual three dimensional space;
- if a user request for flexing the previously represented plane by moving at least one of the points comprised in the represented plane respect to the flexing centre point is detected:
   - representing said previously represented plane being flexed by moving at least one of the points comprised in said previously represented plane respect to the flexing centre point.

Flexing a plane by defining a flexing centre point and dragging at least one of the other control points respect to said flexing centre point also implies that very few intuitive user actions are necessary for that. Then, a further ease-of-use facility for the surgeon is offered and, therefore, performance of robotic surgical operations is highly improved. Moreover, said flexing functionality helps the user adjusting the configuration of planes in the virtual 3D space, so that representations of very high accuracy can be achieved.

In a preferred embodiment of the invention, the method further comprises:
- if a user request for joining at least two of the represented planes is detected:
   - joining said represented planes by limiting their measurements according to the intersections curve segments between said represented planes in the virtual three dimensional space;
and representing the working space limits according to the space being limited by said at least one plane intersecting the three dimensional image of the anatomical element in the virtual three dimensional space comprises:
- representing the working space limits according to the space comprised between said joined represented planes in the virtual three dimensional space.

Representing working space limits from joined planes also implies that very few intuitive user actions are necessary for that. Then, a further ease-of-use facility for the surgeon is offered and, therefore, performance of robotic surgical operations is highly improved. Moreover, said functionality of representing working space limits from joined planes helps the user adjusting the configuration of said limits in the virtual 3D space, so that representations of very high accuracy can be achieved.

In some embodiments, the method further comprises:
- if a user request for rotating the three dimensional image of the anatomical element respect at least one axis of a predetermined three dimensional coordinate system is detected:
   - representing the three dimensional image of the anatomical element being rotated respect the at least one axis of the predetermined three dimensional coordinate system in the virtual three dimensional space.

Rotating the three dimensional image of the anatomical element also implies that very few intuitive user actions are necessary for that. Then, a further ease-of-use facility for the surgeon is offered and, therefore, performance of robotic surgical operations is highly improved. Moreover, said functionality of rotating the three dimensional image helps the user obtaining the optimal orientation of the three dimensional image in the virtual 3D space, so that representations of very high accuracy can be achieved.

In a second aspect, the present invention provides a system for defining working space limits in robotic surgery, wherein a user manages a surgical robot acting on at least one anatomical element, the movements of the surgical robot being to be limited within said working space limits, the system comprising:
- computer means for representing a three dimensional image of the anatomical element in a virtual three dimensional space;
- computer means for representing at least one plane intersecting the three dimensional image of the anatomical element in the virtual three dimensional space;
- computer means for representing the working space limits according to the space being limited by said at least one plane intersecting the three dimensional image of the anatomical element in the virtual three dimensional space.

In a third aspect, the present invention provides a Graphical User Interface for defining working space limits in robotic surgery, wherein a user manages a surgical robot acting on at least one anatomical element, the movements of the surgical robot being to be limited within said working space limits, the Graphical User Interface comprising:
- a first at least one control element wherein a virtual three dimensional space is represented;
- a second at least one control element for executing at least one user request for representing a three dimensional image of the anatomical element in the virtual three dimensional space, said second control element, when selected, generating at least one control signal causing representation of the three dimensional image of the anatomical element in the first control element;
- a third at least one control element for executing at least one user request for representing at least one plane intersecting the three dimensional image of the anatomical element in the virtual three dimensional space, said third control element, when selected, generating at least one control signal causing representation of the at least one plane intersecting the three dimensional image of the anatomical element in the first control element;
- a fourth at least one control element for executing at least one user request for representing the working space limits according to the space being limited by said at least one plane intersecting the three dimensional image of the anatomical element in the virtual three dimensional space, said fourth control element, when selected, generating at least one control signal causing representation of the working space limits according to the space being limited by said at least one plane intersecting the three dimensional image of the anatomical element in the first control element.

According to T-decisions T-0049/04 and T-0643/00, an arrangement of menu items (or images) on a screen may be determined by technical considerations. Such considerations may aim at enabling the user to perform a technical task, such as searching and retrieving images stored in an image processing apparatus, in a more efficient or faster manner, even if an evaluation by the user on a mental level is involved. Although such evaluation per se does not fall within the meaning of "invention" pursuant to Article 52 EPC, the mere fact that mental activities are involved does not necessarily qualify subject matter as non-technical since any technical solutions in the end aim at providing tools which serve, assist or replace human activities of different kinds, including mental ones. Thus, the present invention should be considered technically contributing to the field of robotic surgery because is based on a GUI that enables the user (surgeon) to perform a technical task (robotic surgical operations) in a more efficient or faster manner.

Each one of the control elements comprised in the GUI of the invention is linked to a module or sub-module implementing one of the previously described functionalities of the method of the invention (e.g. representing curve segments, representing planes, rotating/flexing curve segments/planes). The combination of said GUI and method (of the invention) constitutes a very powerful and ease-to-use tool for defining working space limits in robotic surgery, because both control elements and related methods (or parts of the method) are base on very intuitive foundations that makes easier and faster to the user representing the necessary graphics for establishing said working space limits.

In a further aspect of the invention, the present invention relates to a computer program product comprising program instructions for causing a computer to perform the method for defining working space limits in robotic surgery. The invention also relates to such a computer program product embodied on a storage medium (for example, a CD-ROM, a DVD, a USB drive, on a computer memory or on a read-only memory) or carried on a carrier signal (for example, on an electrical or optical carrier signal).

### BRIEF DESCRIPTION OF THE DRAWINGS

Particular embodiments of the present invention will be described in the following, only by way of non-limiting example, with reference to the appended drawings, in which:
Figure 1 is a schematic representation of a three dimensional image of an anatomical element being intersected by a plane defining working space limits in a virtual 3D space, according to the invention;
Figure 2 is a schematic representation of a plane generated from a curve segment generated from several control points in a virtual 3D space, according to the invention;
Figure 3 is a schematic representation of a rotated curve segment in a virtual 3D space, according to the invention;
Figure 4 is a schematic representation of a plane generated from two curve segments in a virtual 3D space, according to the invention;
Figure 5 is a schematic representation of a flexed plane in a virtual 3D space, according to the invention;
Figure 6 is a flow chart of a preferred embodiment of the method of the invention for defining working space limits in robotic surgery, according to the invention;
Figure 7 is a flow chart of the functionality for representing a curve segment in the virtual three dimensional space of a preferred embodiment of the method of the invention for defining working space limits in robotic surgery, according to the invention;
Figure 8 is a flow chart of the functionality for rotating a curve segment in the virtual three dimensional space of a preferred embodiment of the method of the invention for defining working space limits in robotic surgery, according to the invention;
Figure 9 is a flow chart of the functionality for flexing a curve segment in the virtual three dimensional space of a preferred embodiment of the method of the invention for defining working space limits in robotic surgery, according to the invention;
Figure 10 is a flow chart of the functionality for representing a plane by translation of a curve segment in the virtual three dimensional space of a preferred embodiment of the method of the invention for defining working space limits in robotic surgery, according to the invention;
Figure 11 is a flow chart of the functionality for representing a plane from at least two curve segments in the virtual three dimensional space of a preferred embodiment of the method of the invention for defining working space limits in robotic surgery, according to the invention;
Figure 12 is a flow chart of the functionality for representing a plane by replication of a previously represented plane in the virtual three dimensional space of a preferred embodiment of the method of the invention for defining working space limits in robotic surgery, according to the invention; and
Figure 13 is a schematic representation of a preferred embodiment of the graphical user interface of the invention for defining working space limits in robotic surgery, according to the invention.

### DESCRIPTION OF EMBODIMENTS

In the following descriptions, numerous specific details are set forth in order to provide a thorough understanding of the present invention. It will be understood, however, to one skilled in the art, that the present invention may be practiced without some or all of these specific details. In other instances, well known elements have not been described in detail in order not to unnecessarily obscure the present invention. It is also important to note that the accompanying drawings are not drawn to scale.

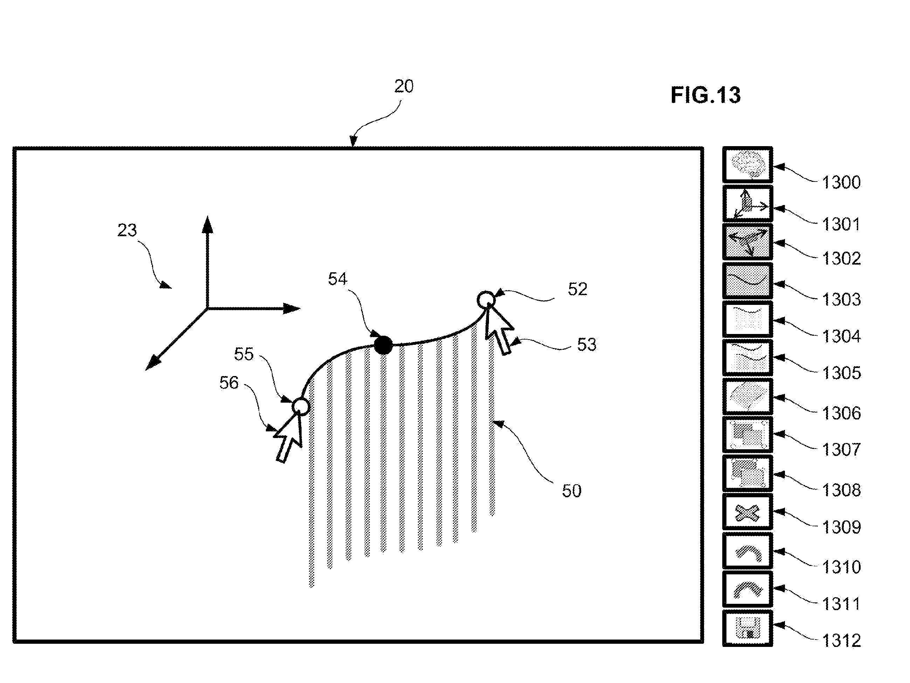

A preferred embodiment of the system of the invention is a computer program implementing the method of the invention for defining working space limits in robotic surgery, wherein a user manages a surgical robot acting on at least one anatomical element, the movements of the surgical robot being to be limited within said working space limits in a virtual three dimensional space comprising a coordinate system of reference. Said computer program comprises a graphical user interface (Figure 13) and several underlying modules connected to said interface, each one of said modules being in charge of several functionalities for representing and manipulating 3D surfaces for defining working space limits in robotic surgery. A first module comprises functionalities for representing three dimensional images of anatomical elements, a second module comprises functionalities for controlling orientation of represented elements, a third module comprises functionalities for representing surfaces, a fourth module comprises functionalities for representing working space limits and a fifth module comprises general functionalities.

The first module for representing three dimensional images of anatomical elements comprises a sub-module for importing three dimensional images of anatomical elements, said sub-module being related to its corresponding option in the GUI.

The second module for controlling orientation comprises a sub-module for fixing orientation of the Z axis and a sub-module for allowing rotation respect to the three axis of the three dimensional coordinate system of reference in the virtual 3D space. Each one of said sub-modules is related to its corresponding option in the GUI.

The third module for representing surfaces comprises a sub-module for representing curve segments, a sub-module for representing planes from a represented curve segment, a sub-module for representing planes from a previously represented plane and a sub-module for representing planes from at least two represented curve segments. Each one of said sub-modules is related to its corresponding option in the GUI.

The fourth module for representing working space limits comprises a sub-module for joining at least two of the represented planes and representing working space limits according to the space comprised between said joined represented planes, and a sub-module for separating planes representing working space limits. Each one of said sub-modules is related to its corresponding option in the GUI.

The fifth module general functionalities comprises a sub-module for deleting any kind of representation (curve segments, planes, etc.), a sub-module for cancelling user actions (undo option), a sub-module for redoing user actions and a sub-module in charge of saving any kind of representation in the virtual 3D space. Each one of said sub-modules is related to its corresponding option in the GUI.

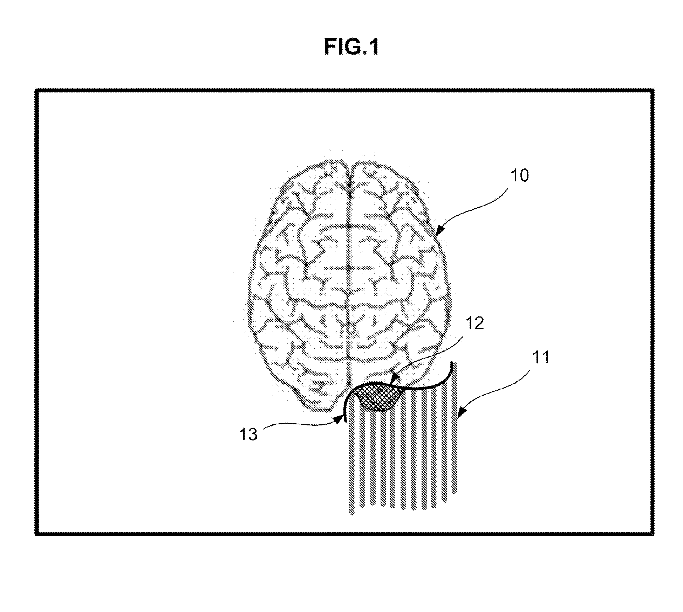

Figure 1 is a schematic representation of a three dimensional image of an anatomical element 10 being intersected by a plane 11 defining working space limits 12 in a virtual 3D space, according to the invention. In said figure 1 the working space limits 12 have been achieved by representing only one plane 11 intersecting the 3D image of a brain 10 in the virtual 3D space, said plane 11 having been generated by translation of a curve segment 13.

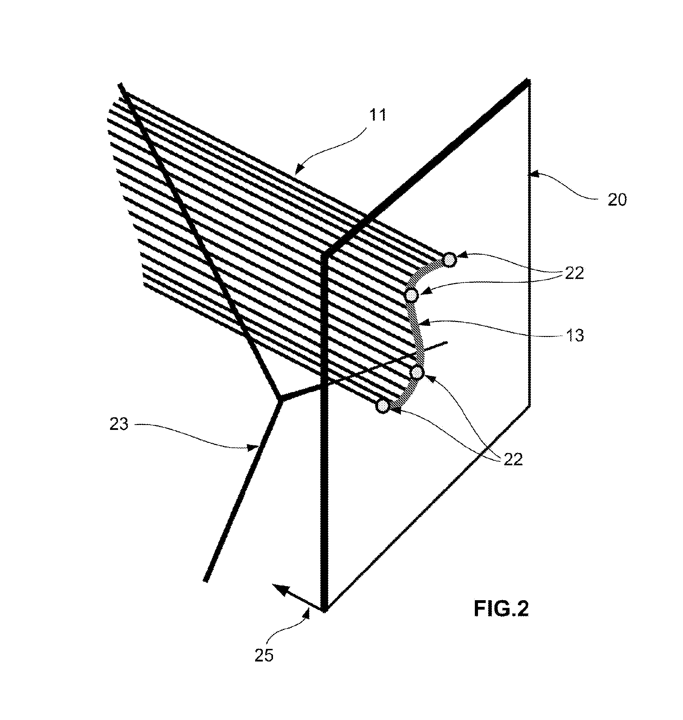

Figure 2 is a schematic representation of a plane generated from a curve segment generated from several control points in a virtual 3D space, according to the invention. In this case, four control points 22 have been represented, from which a curve segment 13 has been obtained. The plane 11 has been generated by translation of the curve segment 13 in a predetermined direction 25 and according to the coordinate system of reference 23. Said representations of control points, curve segment and plane have been generated in the virtual 3D space represented in a particular window 20 of the GUI.

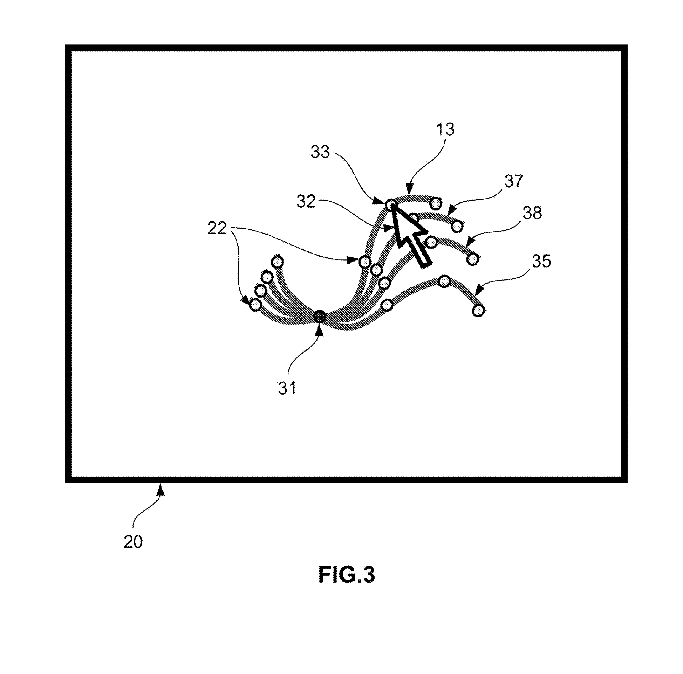

Figure 3 is a schematic representation of a rotated curve segment in a virtual 3D space, according to the invention. First of all, a curve segment 13 is represented by previously defining several control points 22, 31, 33, the curve segment 13 being obtained through the option of representing curve segments by polynomial interpolation of control points 22, 31, 33. Secondly, one of the control points 22, 31, 33 has been defined as rotation centre point 31. And finally, the curve segment 13 is rotated respect the rotation centre point 31 by dragging the control point 33 through the pointer 32 that may respond to, for example, the movement of a traditional mouse. As can be seen, the rotation may produce any of the curve segments 37, 38, 35. Said representations have been generated in the virtual 3D space represented in a particular window 20 of the GUI.

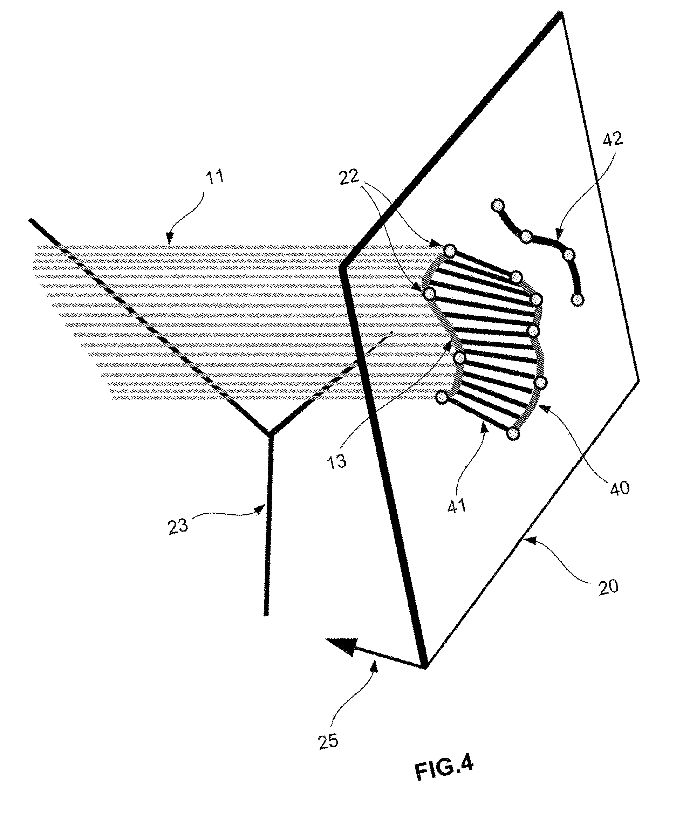

Figure 4 is a schematic representation of a plane generated from two curve segments in a virtual 3D space, according to the invention. First, two curve segments 13, 40 have been generated from the corresponding control points 22. Second, a plane 41 configured by said two curve segments 13, 40 has been represented by parametric extension of said at least two curve segments 13, 40. The figure also shows another possible curve segment 42, from which other planes may be generated, another possible plane 11, which has been defined by translation of the curve segment 13 in the predetermined direction 25 and according to the coordinate system of reference 23. Said representations have been generated in the virtual 3D space represented in a particular window 20 of the GUI.

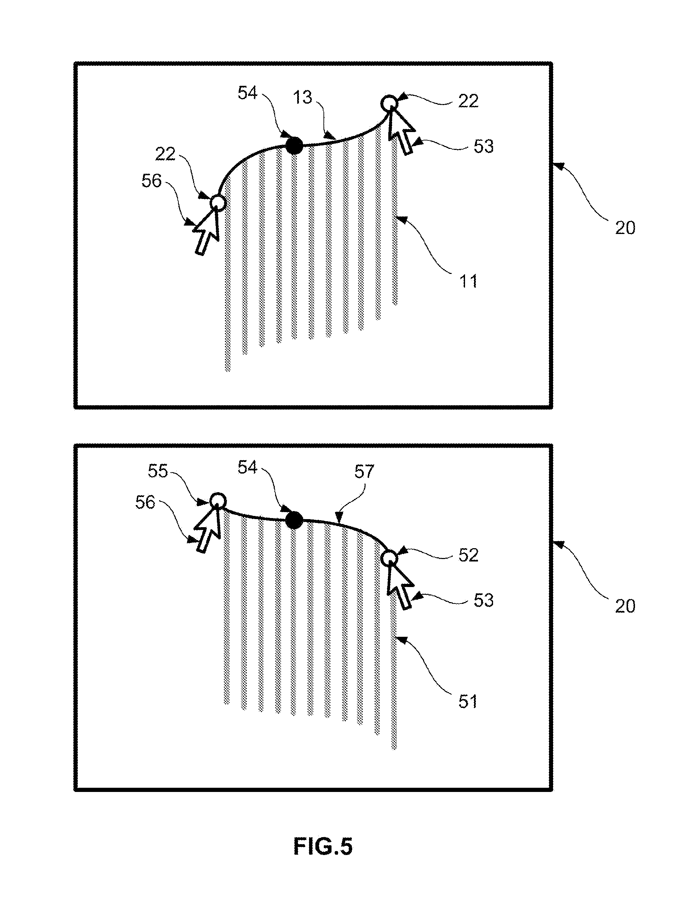

Figure 5 is a schematic representation of a flexed plane in a virtual 3D space, according to the invention. The plane 11 has been configured from the curve segment 13, which has been derived from the control points 22, 54. One of said control points has been defined as a flexing centre point 54 in order to flex the plane 11 by dragging the control points 22 respect to the flexing centre point 54. Said dragging operation may be done by using two mouse or similar devices, so that the control points 22 are dragged according the movement of two pointers 53, 56 related to the movement of the mouse. It is finally shown the result of said flexing operation: the plane 51, which is defined according the new positions 52, 55 of the dragged control points 22. Said representations have been generated in the virtual 3D space represented in a particular window 20 of the GUI.
   In relation to the figure 5, it has to be taken into account that said figure may also serve to understand how flexion of a curve segment 13 works, since it is only a matter of eliminating the represented plane 11 and flexed plane 51. With this elimination, flexion of the curve segment 13 is also perfectly explained.

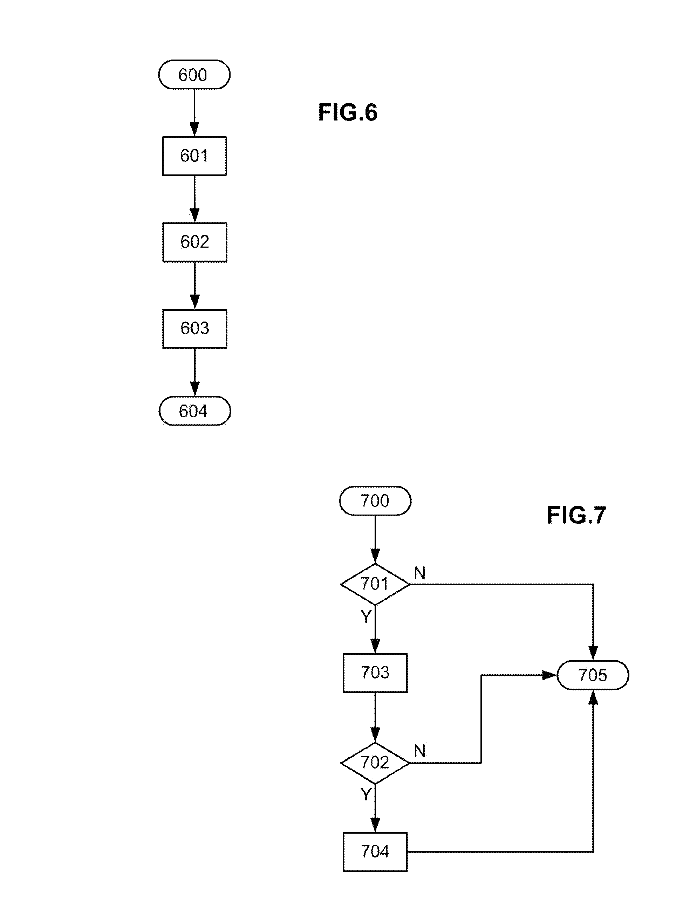

Figure 6 is a flow chart of a preferred embodiment of the method of the invention for defining working space limits in robotic surgery, according to the invention. Said preferred embodiment comprises:
   - starting 600 execution;
   - representing 601 a three dimensional image of the anatomical element 10 in a virtual three dimensional space;
   - representing 602 at least one plane 11 intersecting the three dimensional image of the anatomical element 10 in the virtual three dimensional space;
   - representing 603 the working space limits 12 according to the space being limited by said at least one plane 11 intersecting the three dimensional image of the anatomical element 10 in the virtual three dimensional space.
   - ending 604 execution.
Figure 7 is a flow chart of the functionality for representing a curve segment in the virtual three dimensional space of a preferred embodiment of the method of the invention for defining working space limits in robotic surgery, according to the invention. This functionality for representing a curve segment comprises:
   - starting 700 execution;
   - if a user request 701 for representing a plurality of control points 22 in the virtual three dimensional space is detected:
      o representing 703 the plurality of control points 22 in the virtual three dimensional space according to the user request 701 for representing the plurality of control points 22;
      o if a user request 702 for representing said curve segment 13 from the plurality of control points 22 in the virtual three dimensional space is detected:
         ■ representing 704 said curve segment 13 by polynomial interpolation of the plurality of control points 22 in the virtual three dimensional space according to the user request 702 for representing said curve segment 13 from the plurality of control points 22.
      o if user request 702 not detected:
         ■ ending 705 execution.
   - if user request 701 not detected:
      o ending 705 execution.

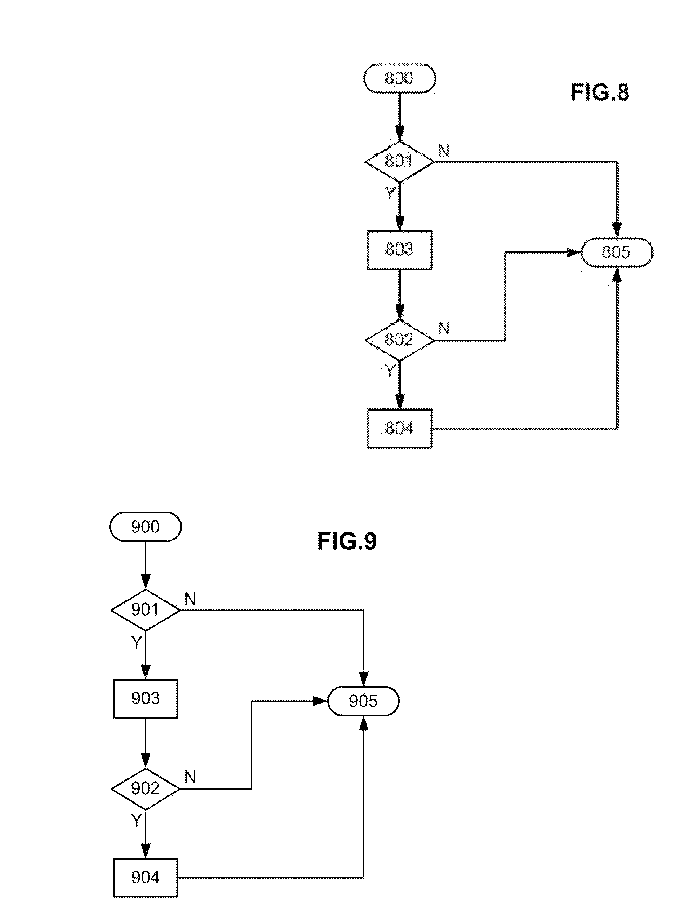

Figure 8 is a flow chart of the functionality for rotating a curve segment in the virtual three dimensional space of a preferred embodiment of the method of the invention for defining working space limits in robotic surgery, according to the invention. Said functionality for rotating a curve segment comprising:
   - starting 800 execution;
   - if a user request 801 for representing at least one of the control points 22, 31, 33 as a rotation centre point 31 in the virtual three dimensional space is detected:
      o representing 803 the rotation centre point 31 in the virtual three dimensional space according to the user request 801 for representing the control point 31 as the rotation centre point 31;
      o if a user request 802 for rotating the curve segment 13 by moving one of the control points 33 respect to the rotation centre point 31 is detected:
         ■ representing 804 the curve segment 13 being rotated 35 by moving one of the control points 33 respect to the rotation centre point 31 according to the user request 802 for rotating the curve segment 13.
      o if user request 802 not detected:
         ■ ending 805 execution.
   - if user request 801 not detected:
      o ending 805 execution.
Figure 9 is a flow chart of the functionality for flexing a curve segment in the virtual three dimensional space of a preferred embodiment of the method of the invention for defining working space limits in robotic surgery, according to the invention. This functionality for flexing a curve segment comprises:
   - starting 900 execution;
   - if a user request 901 for representing at least one of the control points 54 as a flexing centre point 54 in the virtual three dimensional space is detected:
      o representing 903 the flexing centre point 54 in the virtual three dimensional space according to the user request 901 for representing the control point 54 as the flexing centre point 54;
      o if a user request 902 for flexing the curve segment 13 by moving at least one of the control points 22 respect to the flexing centre point 54 is detected:
         ■ representing 904 the curve segment 13 being flexed 57 by moving at least one of the control points 22 respect to the flexing centre point 54 according to the user request 902 for flexing the curve segment 13.
      o if user request 902 not detected:
         ■ ending 905 execution.
   - if user request 901 not detected:
      o ending 905 execution.

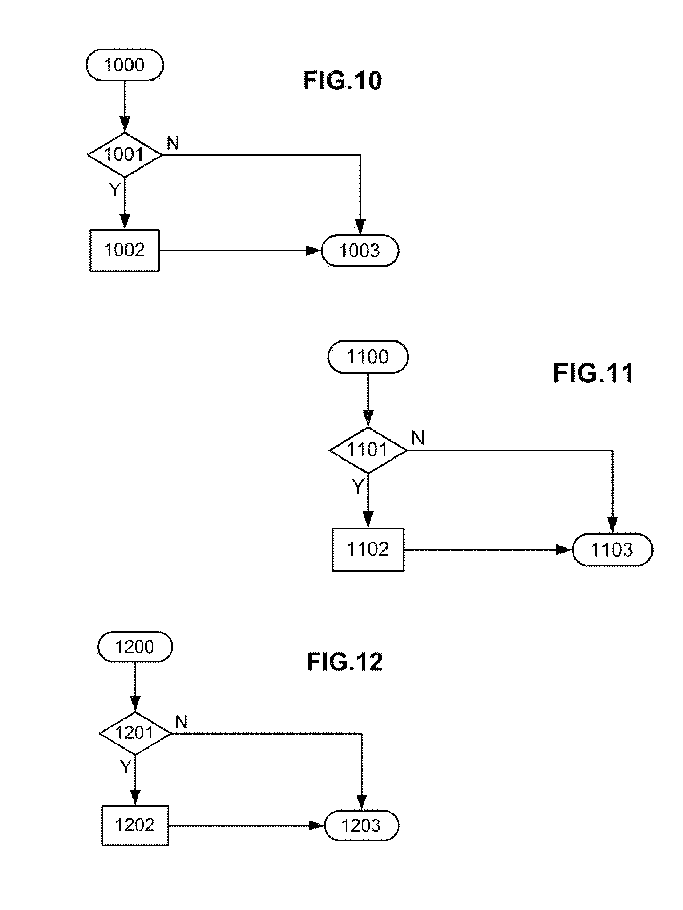

Figure 10 is a flow chart of the functionality for representing a plane by translation of a curve segment in the virtual three dimensional space of a preferred embodiment of the method of the invention for defining working space limits in robotic surgery, according to the invention. Said functionality for representing a plane by translation of a curve segment comprises:
   - if a user request 1001 for representing at least one of the planes 11 intersecting the three dimensional image of the anatomical element 10 by translation of said at least one curve segment 13 in the virtual three dimensional space is detected:
      o representing 1002 said one of the planes 11 intersecting the three dimensional image of the anatomical element 10 by translation in a predetermined direction of said curve segment 13 in the virtual three dimensional space;
   - ending 1003 execution.
Figure 11 is a flow chart of the functionality for representing a plane from at least two curve segments in the virtual three dimensional space of a preferred embodiment of the method of the invention for defining working space limits in robotic surgery, according to the invention. This functionality for representing a plane from at least two curve segments comprises:
   - if a user request 1101 for representing at least one of the planes 41 intersecting the three dimensional image of the anatomical element 10 from at least two curve segments 13, 40 of the at least one curve segment in the virtual three dimensional space:
      o representing 1102 said one of the planes 41 intersecting the three dimensional image of the anatomical element 10 by parametric extension of said at least two curve segments 13, 40 of the at least one curve segment in the virtual three dimensional space;
   - ending 1103 execution.
Figure 12 is a flow chart of the functionality for representing a plane by replication of a previously represented plane in the virtual three dimensional space of a preferred embodiment of the method of the invention for defining working space limits in robotic surgery, according to the invention. Said functionality for representing a plane by replication of a previously represented plane comprising:
   - if a user request 1201 for representing at least one of the planes intersecting the three dimensional image of the anatomical element 10 from a previously represented plane 11 in the virtual three dimensional space is detected:
      o representing 1202 said one of the planes intersecting the three dimensional image of the anatomical element 10 by replication of said previously represented plane 11 in the virtual three dimensional space;
   - ending 1103 execution.
Figure 13 is a schematic representation of a preferred embodiment of the graphical user interface GUI of the invention for defining working space limits in robotic surgery, according to the invention. Said GUI comprises:
   a window 20 wherein a virtual three dimensional space is represented;
   a button 1300 for executing at least one user request for representing 601 a three dimensional image of the anatomical element 10 in the virtual three dimensional space, said button 1300, when selected, generating at least one control signal causing representation 601 of the three dimensional image of the anatomical element 10 in the window 20;
   two buttons 1301, 1302 for controlling orientation of represented elements in the virtual three dimensional space, said buttons 1301, 1302, when selected, generating at least one control signal causing control of orientation of represented elements in the virtual three dimensional space represented in window 20;
   four buttons 1303, 1304, 1305, 1306 for executing at least one user request for representing 602 at least one plane 11 intersecting the three dimensional image of the anatomical element 10 in the virtual three dimensional space, said four buttons 1303, 1304, 1305, 1306, when selected, generating at least one control signal causing representation 602 of the at least one plane 11 intersecting the three dimensional image of the anatomical element 10 in window 20;
   two buttons 1307, 1308 for executing at least one user request for representing 603 the working space limits 12 according to the space being limited by said at least one plane 11 intersecting the three dimensional image of the anatomical element 10 in the virtual three dimensional space, said two buttons 1307, 1308, when selected, generating at least one control signal causing representation 603 of the working space limits 12 according to the space being limited by said at least one plane 11 intersecting the three dimensional image of the anatomical element 10 in window 20; and
   four buttons for general functionalities: deleting 1309 any kind of representation (curve segments, planes, etc.), cancelling 1310 user actions undo option, redoing 1311 user actions and saving 1312 any kind of representation in the virtual 3D space.

Each one of the above mentioned buttons is related to its corresponding sub-module for performing its associated functionality. For example, button 1303 is linked to the sub-module for representing curve segments, button 1304 is associated with the sub-module for representing planes from a represented curve segment, button 1305 is related to the sub-module for representing a plane from a previously represented plane and button 1306 is linked to the sub-module for representing planes from at least two represented curve segments.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. Thus, it is intended that the scope of the present invention herein disclosed should not be limited by the particular disclosed embodiments described before, but should be determined only by a fair reading of the claims that follow.

## Claims

1. A method for defining working space limits (12) in robotic surgery, wherein a user manages a surgical robot acting on at least one anatomical element (10), the movements of the surgical robot being to be limited within said working space limits (12), the method comprising:
• representing (601) a three dimensional image of the anatomical element (10) in a virtual three dimensional space;
• representing (602) at least one plane (11) intersecting the three dimensional image of the anatomical element (10) in the virtual three dimensional space;
• representing (603) the working space limits (12) according to the space being limited by said at least one plane (11) intersecting the three dimensional image of the anatomical element (10) in the virtual three dimensional space.

2. The method according to claim 1, wherein representing at least one plane (11) intersecting the three dimensional image of the anatomical element (10) in the virtual three dimensional space comprises:
• if a user request (701, 702; 801, 802; 901, 902) for representing at least one curve segment (13) in the virtual three dimensional space is detected:
• representing (703, 704; 803, 804; 903, 904) said at least one curve segment (13) in the virtual three dimensional space according to the user request (701, 702; 801, 802; 901, 902) for representing said at least one curve segment (13);
• if a user request (1001; 1101) for representing at least one of the planes (11; 41) intersecting the three dimensional image of the anatomical element (10) from said at least one curve segment (13; 40) in the virtual three dimensional space is detected:
• representing (1002; 1102) said one of the planes (11; 41) intersecting the three dimensional image of the anatomical element (10) from said at least one curve segment (13; 40) in the virtual three dimensional space.

3. The method according to claim 2, wherein the user request (701, 702; 801, 802; 901, 902) for representing at least one curve segment (13) in the virtual three dimensional space comprises:
• a user request (701) for representing a plurality of control points (22) in the virtual three dimensional space;
• a user request (702) for representing said curve segment (13) from the plurality of control points (22) in the virtual three dimensional space;
and wherein representing (703, 704; 803, 804; 903, 904) said at least one curve segment (13) in the virtual three dimensional space according to the user request (701, 702; 801, 802; 901, 902) for representing said at least one curve segment (13) comprises:
• representing (703) the plurality of control points (22) in the virtual three dimensional space according to the user request (701) for representing the plurality of control points (22);
• representing (704) said curve segment (13) by polynomial interpolation of the plurality of control points (22) in the virtual three dimensional space according to the user request (702) for representing said curve segment (13) from the plurality of control points (22).

4. The method according to claim 3, wherein the user request (701, 702; 801, 802; 901, 902) for representing at least one curve segment (13) in the virtual three dimensional space comprises:
• if the user request (701, 702; 801, 802; 901, 902) refers to rotation (801, 802) of said curve segment (13):
• a user request (801) for representing at least one of the control points (22, 31, 33) as a rotation centre point (31) in the virtual three dimensional space;
• a user request (802) for rotating the curve segment (13) by moving one of the control points (33) respect to the rotation centre point (31);
and wherein representing (703, 704; 803, 804; 903, 904) said at least one curve segment (13) in the virtual three dimensional space according to the user request (701, 702; 801, 802; 901, 902) for representing said at least one curve segment (13) comprises:
• if the user request (701, 702; 801, 802; 901, 902) refers to rotation (801, 802) of said curve segment (13):
• representing (803) the rotation centre point (31) in the virtual three dimensional space according to the user request (801) for representing the control point (31) as the rotation centre point (31);
• representing (804) the curve segment (13) being rotated (35) by moving one of the control points (33) respect to the rotation centre point (31) according to the user request (802) for rotating the curve segment (13).

5. The method according to any of claims 3 or 4, wherein the user request (701, 702; 801, 802; 901, 902) for representing at least one curve segment (13) in the virtual three dimensional space comprises:
• if the user request (701, 702; 801, 802; 901, 902) refers to flexion (901, 902) of said curve segment (13):
• a user request (901) for representing at least one of the control points (54) as a flexing centre point (54) in the virtual three dimensional space;
• a user request (902) for flexing the curve segment (13) by moving at least one of the control points (22) respect to the flexing centre point (54);
and wherein representing (703, 704; 803, 804; 903, 904) said at least one curve segment (13) in the virtual three dimensional space according to the user request (701, 702; 801, 802; 901, 902) for representing said at least one curve segment (13) comprises:
• if the user request (701, 702; 801, 802; 901, 902) refers to flexion (901, 902) of said curve segment (13):
• representing (903) the flexing centre point (54) in the virtual three dimensional space according to the user request (901) for representing the control point (54) as the flexing centre point (54);
• representing (904) the curve segment (13) being flexed (57) by moving at least one of the control points (22) respect to the flexing centre point (54) according to the user request (902) for flexing the curve segment (13).

6. The method according to any of claims 3 to 5, wherein the user request (1001; 1101) for representing at least one of the planes (11; 41) intersecting the three dimensional image of the anatomical element (10) from said at least one curve segment (13; 40) in the virtual three dimensional space comprises:
• if the user request (1001; 1101) refers to representation of plane (11) by translation (1001) of curve segment (13):
• a user request (1001; 1101) for representing said one of the planes intersecting the three dimensional image of the anatomical element (10) by translation (1001) in a predetermined direction of said curve segment (13) in the virtual three dimensional space;
and wherein representing (1002; 1102) said one of the planes (11; 41) intersecting the three dimensional image of the anatomical element (10) from said at least one curve segment (13; 40) in the virtual three dimensional space comprises:
• if the user request (1001; 1101) refers to representation of plane (11) by translation (1001) of curve segment (13):
• representing (1002) said one of the planes (11) intersecting the three dimensional image of the anatomical element (10) by translation in a predetermined direction of said curve segment (13) in the virtual three dimensional space.

7. The method according to any of claims 3 to 6, wherein the user request (1001; 1101) for representing at least one of the planes (11; 41) intersecting the three dimensional image of the anatomical element (10) from said at least one curve segment (13; 40) in the virtual three dimensional space comprises:
• if the user request (1001; 1101) refers to representation (1101) of plane (41) from at least two curve segments (13, 40):
• a user request (1101) for representing said one of the planes (41) intersecting the three dimensional image of the anatomical element (10) from at least two curve segments (13, 40) of the at least one curve segment in the virtual three dimensional space;
and wherein representing (1002; 1102) said one of the planes (11; 41) intersecting the three dimensional image of the anatomical element (10) from said at least one curve segment (13; 40) in the virtual three dimensional space comprises:
• if the user request (1001; 1101) refers to representation (1101) of plane (41) from at least two curve segments (13, 40):
• representing said one of the planes (41) intersecting the three dimensional image of the anatomical element (10) by parametric extension of said at least two curve segments (13, 40) of the at least one curve segment in the virtual three dimensional space.

8. The method according to any of claims 1 to 7, wherein representing (602) at least one plane (11) intersecting the three dimensional image of the anatomical element (10) in the virtual three dimensional space comprises:
• if a user request (1201) for representing at least one of the planes intersecting the three dimensional image of the anatomical element (10) from a previously represented plane (11) in the virtual three dimensional space is detected:
• representing (1202) said one of the planes intersecting the three dimensional image of the anatomical element (10) by replication of said previously represented plane (11) in the virtual three dimensional space.

9. The method according to any of claims 1 to 8, wherein representing (602) at least one plane (11) intersecting the three dimensional image of the anatomical element (10) in the virtual three dimensional space comprises:
• if a user request for representing a point comprised in a previously represented plane (11) as a rotation centre point of the plane in the virtual three dimensional space is detected:
• representing said point comprised in the previously represented plane (11) as a rotation centre point of the plane (11) in the virtual three dimensional space;
• if a user request for rotating the previously represented plane (11) by moving at least one of the points comprised in the previously represented plane (11) respect to the rotation centre point is detected:
• representing said previously represented plane (11) being rotated by moving at least one of the points comprised in said previously represented plane (11) respect to the rotation centre point.

10. The method according to any of claims 1 to 9, wherein representing (602) at least one plane (11) intersecting the three dimensional image of the anatomical element (10) in the virtual three dimensional space comprises:
• if a user request for representing a point (54) comprised in a previously represented plane (11) as a flexing centre point (54) of the plane (11) in the virtual three dimensional space is detected:
• representing said point (54) comprised in the previously represented plane (11) as a flexing centre point (54) of the plane (11) in the virtual three dimensional space;
• if a user request for flexing the previously represented plane (11) by moving at least one of the points (22) comprised in the represented plane respect to the flexing centre point (54) is detected:
• representing said previously represented plane (11) being flexed (51) by moving at least one of the points (22) comprised in said previously represented plane (11) respect to the flexing centre point (54).

11. The method according to any of claims 1 to 10, further comprising:
• if a user request for joining at least two of the represented planes (11; 41) is detected:
• joining said represented planes (11; 41) by limiting their measurements according to the intersections curve segments (13) between said represented planes (11; 41) in the virtual three dimensional space;
and wherein representing the working space limits (12) according to the space being limited by said at least one plane (11; 41) intersecting the three dimensional image of the anatomical element (10) in the virtual three dimensional space comprises:
• representing the working space limits (12) according to the space comprised between said joined represented planes (11; 41) in the virtual three dimensional space.

12. The method according to any of claims 1 to 11, further comprising:
• if a user request for rotating the three dimensional image of the anatomical element (10) respect at least one axis of a predetermined three dimensional coordinate system (23) is detected:
• representing the three dimensional image of the anatomical element (10) being rotated respect the at least one axis of the predetermined three dimensional coordinate system (23) in the virtual three dimensional space.

13. A system for defining working space limits (12) in robotic surgery, wherein a user manages a surgical robot acting on at least one anatomical element (10), the movements of the surgical robot being to be limited within said working space limits (12), the system comprising:
• computer means for representing (601) a three dimensional image of the anatomical element (10) in a virtual three dimensional space;
• computer means for representing (602) at least one plane (11) intersecting the three dimensional image of the anatomical element (10) in the virtual three dimensional space;
• computer means for representing (603) the working space limits (12) according to the space being limited by said at least one plane (11) intersecting the three dimensional image of the anatomical element (10) in the virtual three dimensional space.

14. A Graphical User Interface for defining working space limits (12) in robotic surgery, wherein a user manages a surgical robot acting on at least one anatomical element (10), the movements of the surgical robot being to be limited within said working space limits (12), the Graphical User Interface comprising:
• a first at least one control element (20) wherein a virtual three dimensional space is represented;
• a second at least one control element (1300) for executing at least one user request for representing (601) a three dimensional image of the anatomical element (10) in the virtual three dimensional space, said second control element (1301; 1302), when selected, generating at least one control signal causing representation (601) of the three dimensional image of the anatomical element (10) in the first control element (20);
• a third at least one control element (1303; 1304; 1305; 1306) for executing at least one user request for representing (602) at least one plane (11) intersecting the three dimensional image of the anatomical element (10) in the virtual three dimensional space, said third control element (1303; 1304; 1305; 1306), when selected, generating at least one control signal causing representation (602) of the at least one plane (11) intersecting the three dimensional image of the anatomical element (10) in the first control element (20);
• a fourth at least one control element (1307; 1308) for executing at least one user request for representing (603) the working space limits (12) according to the space being limited by said at least one plane (11) intersecting the three dimensional image of the anatomical element (10) in the virtual three dimensional space, said fourth control element (1307; 1308), when selected, generating at least one control signal causing representation (603) of the working space limits (12) according to the space being limited by said at least one plane (11) intersecting the three dimensional image of the anatomical element (10) in the first control element (20).

15. A computer program product comprising program instructions for causing a computer to perform the method for defining working space limits (12) in robotic surgery, according to any of claims 1 to 12.

16. A computer program product according to claim 15, embodied on storing means.

17. A computer program product according to claim 15, carried on a carrier signal.
